# EUROPEAN PATENT APPLICATION

(11) **EP 3 772 538 A1**
(43) Date of publication of application: **10.02.2021**
(21) Application number: 19290013.2
(22) Date of filing: 09.08.2019
(51) Int. Cl.: C12N 5/071, C12N 5/09, G01N 33/50

(54) **METHOD FOR DIFFERENTIATING EPITHELIAL STEM CELLS**

(71) Applicant: Urosphere, 31400 Toulouse (FR); Institut National de la Santé et de la Recherche Médicale, 75013 Paris (FR); Université Toulouse III-Paul Sabatier, 31062 Toulouse Cedex 9 (FR); Ecole Nationale Vétérinaire de Toulouse, 31300 Toulouse (FR); Chu Rangueil, 31059 Toulouse (FR)
(72) Inventor: Lluel, Philippe, 31400 Toulouse (FR); Vergnolle, Nathalie, 82500 Le Cause (FR); Game, Xavier, 31400 Toulouse (FR)
(74) Representative: Ernest Gutmann - Yves Plasseraud S.A.S.

(57) **Abstract**

The subject matter of the present invention is a method for differentiating epithelial stem cells, comprising culturing one or more epithelial stem cells in contact with an extracellular matrix in the presence of an expansion medium, a bovine pituitary extract, a receptor tyrosine kinase ligand, a supernatant of primary fibroblasts and optionally, a Rho kinase inhibitor.

## Description

The subject matter of the present invention is a method for differentiating epithelial stem cells, comprising culturing one or more epithelial stem cells in contact with an extracellular matrix in the presence of an expansion medium, a bovine pituitary extract, a receptor tyrosine kinase ligand, a supernatant of primary fibroblasts, and optionally, a Rho kinase inhibitor.

The subject of the invention is also a method that further comprises obtaining and isolating an organoid.

Another subject of the invention is the use of this organoid or a cell derived from said organoid, in a drug discovery development; drug screen; target screen; biomarker screen; toxicity assay; gene expression studies including recombinant gene expression; research of mechanisms involved in tissue injury and repair; research of inflammatory and infectious diseases; research on the microbiota and associated dysbiosis and pathologies; research on all epithelial dysfunction, studies of pathogenic mechanisms; or studies of mechanisms of cell transformation and aetiology of cancer and precision medicine.

Bladder related diseases are among the most common and costly diseases. In particular, Bladder cancer is the tenth most common cancer worldwide and the second most common urologic cancer after prostate cancer (GLOBOCAN 2018). Men are more affected than women (3.2:0.9 ratio) and disease incidence increases with age (Sanli et al., Nature Reviews Disease Primers, 2017; vol 3, p17022). Thus, the disease ranks higher among men, in whom it is the sixth most common cancer and ninth leading cause of cancer death (Bray et al., Ca Cancer J Clin 2018; 68:394-424). Its incidence is over increasing by 1% each year and yet mortality has remained stable over the past 30 years (Rébillard et al., Progrès en Urologie J. Assoc Francaise Urol, 2010 S4:S211-214).

Three main risk factors have been identified in the development of bladder tumours, including smoking, genetic factors, and environmental factors (Sanli *et al.,* 2017). To develop new treatments, the improvement and development of new models for the preclinical study of bladder urothelium is mandatory.

Currently used strategies for disease characterization and therapy development rely on 2D cell culture techniques with subsequent employment of animal models. The *in vitro* models available routinely for the study of the bladder cancer are essentially tumor cell lines grown in 2D. These tumor lines are easy to handle experimentally, are inexpensive, provide extensive information on cellular pathophysiology and have proven utility in preclinical pharmacology studies. However, these models of 2D tumor lines do not recreate tissue architecture and environmental diversity, nor the difficulties of therapeutic targeting found *in vivo.* In addition, these cell lines do not reflect tumor heterogeneity between patients and within the tumor itself. Indeed, the cell lines do not allow to faithfully reproduce the genetic and phenotypic diversity of primary tumours and do not fully summarize their complexity because the inflammatory components and stroma are not present *in vitro* (Mo Li et al, the New England Journal of Medicine, 2019; 380(6):569-579).

Animal models are one of the main tools of cancer research. There are currently three mouse models of bladder cancers: chemically induced tumor models, xenograft models and genetically engineered models (GEM) (Inoue et al., Nature Reviews Urology, 2017). Chemical carcinogens models of tumour and their temporal progression are highly heterogeneous whereas GEM models are relatively genetically homogeneous compared with the heterogeneity of human cancers with the same histology. Thus, these models do not accurately recapitulate the tumour. Conventional cell-line derived xenograft (CDX) models have provided valuable information regarding our understanding of cancer development and metastasis. They have been used for drugs screening and understanding of their mechanism of action. But they have major deficiencies as inappropriate microenvironments and the lack of tumor heterogeneity that are necessary for the study of tumour development and resistance. Patient-derived-xenograft models (PDX) are more indicated than CDX to study the tumour biology as they better reflect the tumour heterogeneity of individual cancers. More, treatment of PDXs reproduce clinical outcomes observed in the individual patient donors (Byrne et al., Nature Reviews in Cancer, 2017). However, the animal pathophysiology makes delicate the clinical extrapolation of therapeutic tests. In addition, the high costs and the time required to use these models hinder the implementation of large-scale therapeutic tests covering the spectrum of vesical carcinomas in humans.

Other models of preclinical cancer studies are emerging, taking into account both the investigative flexibility of *in vitro* models and critical elements of *in vivo* physiopathology such as architecture and tissue microenvironment. These are 3D tumor cell cultures forming spheroids. Among these cultures in 3D, the organoid model seems to be the most relevant for the study of cancers. Indeed, organoids can be developed from primary cells isolated from patients, thanks to the survival and self-renewal properties of stem cells, and can differentiate in respect of hierarchy and tissue architecture (Fatehullah et al., Nature Cell Biology, 2016, 18(3):246-254). The organoids closely resemble the *in vivo* epithelial tissue from which they were obtained. They reproduce the complex spatial morphology of a differentiated epithelium to enable biologically relevant cell-cell and cell-matrix interactions. Ideally, the physical, cellular, and molecular characteristics of organoids mean that they share similar physiological responses with differentiated *in vivo* epithelia. Organoid culture was established for several tissue types: stomach, colon and pancreas and for prostate, saliva glands and several other tissues (Mo Li et al., the New England Journal of Medicine, 2019, 380(6):569-579).

Increasing evidence suggests that bladder urothelium is a hierarchically organized tissue containing specific stem cells (SCs) that participate in normal homeostasis and response in bladder aggression. Also, in bladder cancers, there would be cancer stem cells (CSCs), having the ability to self-renew and produce tumor cell heterogeneity by differentiating. Since bladder cancer stem cells are proposed as the drivers of initiation and progression of urothelial cancers, these cells are ideal targets for anticancer therapies. The organoid model would therefore be particularly suitable for the study of normal and cancerous bladder cancer and the development of new treatments.

One of the expected perspectives is to define a new preclinical model opening the door of personalized medicine towards which current research tends with the possibility of *in vitro* tests for the prediction of therapeutic responses. The development of these models can also serve as a support for pathophysiological studies of vesical urothelial diseases (overactive bladder, interstitial cystitis, chemical cystitis, neurogenic bladder, bladder outlet obstruction, underactive bladder, bladder inflammation...) and cellular therapies. Bladder cancer generally originates from the epithelium (urothelium) of the bladder and is referred to as urothelial carcinoma. Non-Muscle Invasive Bladder Cancers that are confined to mucosa or have invaded the lamina propria (submucosa) are classified as Ta and Tl, respectively (Babjuk et al., European Urology, 2017, 71:447-461). Carcinoma *in situ* is a flat, poorly differentiated tumour confined to mucosa. Stage 2 tumours have invaded the muscle layer either superficially (T2a) or deeply (T2b). T3 tumours have invaded beyond the bladder wall into perivesical fat (T3a invasion is microscopic, T3b is macroscopic). T4a tumours have invaded the prostate, uterus, vagina and/or bowel, whereas T4b tumours have invaded the pelvic or abdominal walls (Leow et al., World Journal of Urology, 2019).

Patient-derived organoids (PDOs) have recently emerged as robust preclinical models for their abilities to recapitulate patient drug responses in the clinic, and they might therefore be integrated into precision medicine programs. Bladder cancer organoids have been developed but most of the models obtained are from non-muscle invasive forms of bladder cancer called papillary tumours (with a success rate of 80%) whereas the success rate for preparing organoids from non-papillary forms or muscle invasive tumours is less than 30% (Yoshida et al., Investigative and clinical urology, 2018; 59:149-151). More, it has been observed by Lee et al (cell, 2018; 173:515-528) that 64% of the organoid lines they developed switched molecular subtype from that of their parental tumour.

In the present invention, inventors have developed a protocol allowing the development of organoids from normal bladder and bladder cancer including muscle invasive bladder cancer (MIBC). Indeed, isolated urothelial stem cells needed a fibroblast secretion medium to be able to form organoid structures in a reproducible manner.

It is, therefore, an object of the present invention to provide a method, allowing the culture of organoids in a reproducible and controlled manner either from a normal organ or from a pathogenic organ like a cancer organ. More precisely, an object of the present invention is to provide a method, allowing the culture of bladder organoids in a reproducible and controlled manner either from a normal bladder or a pathogenic bladder like a cancer bladder.

Here is provided a method to culture epithelial stem cells and to obtain organoids that show longer-lived maintenance, and are able to differentiate to all major differentiated cell lineages present in the corresponding *in vivo* tissue. The method is envisaged to be relevant to the culture of all epithelial cell types, in particular bladder cell type.

In particular, the invention provides a method for culturing epithelial stem cells, wherein said method comprises culturing one or more epithelial stem cells in contact with an extracellular matrix in the presence of an expansion medium as described herein.

In some embodiments, the method for culturing or differentiating epithelial stem cells further comprises isolating one or more adult stem cells or obtaining and isolating an organoid. In some embodiments, the epithelial stem cells are from the bladder.

Also provided is a culture medium. In particular, the invention provides an expansion medium, comprising a basal medium for animal or human cells to which is added a bovine pituitary extract (BPE), a receptor tyrosine kinase ligand, a supernatant of primary fibroblasts, and optionally, a Rho kinase inhibitor (rho-associated protein kinase inhibitor or ROCK inhibitor).

The invention also provides an organoid obtainable by the method of culture of the invention. Uses of the organoids described herein and cells derived from the organoids are likewise provided. For example, the invention also provides the use of an organoid of the invention or a cell derived from said organoid in a drug discovery development; drug screen; target screen; biomarker screen; toxicity assay; gene expression studies including recombinant gene expression; research of mechanisms involved in tissue injury and repair; research of inflammatory and infectious diseases; research on the microbiota and associated dysbiosis and pathologies; research on all epithelial dysfunctions, studies of pathogenic mechanisms; or studies of mechanisms of cell transformation, aetiology of cancer and precision medicine. By precision medicine is meant a tool to determine the right treatment at the right moment for a patient but also the possibility to optimize patient recruitment for clinical trials by using an avatar that can be a PDX or an organoid, based on the latter responses to selected drugs or biomarkers expression.

The invention also provides an organoid of the invention, or a cell derived from said organoid, for use in treating a disorder, condition or disease.

The invention also provides an organoid of the invention, or a cell derived from said organoid for use in regenerative medicine, for example, wherein the use involves transplantation of the organoid or cell into a patient.

Other subjects will emerge from reading of the description, the examples and the figures which follow.
Figures 1 and 2 describe cultures of healthy bladder organoids in presence of different culture media. a) Medium KSFM + BPE + hEGF magnitude 10x, b) medium KSFM + BPE + hEGF + fibroblast supernatant, magnitude 20x.
Figure 3 describes tumoural organoids, magnitude 10x. a) bilobed organoids, b) organoids in cluster.
Figures 4 and 5 show a morphological comparison between healthy c) and tumoural d) organoids at D15, magnitude 10x.
Figure 6 describes the IF characterization of organoids from a healthy bladder 1 actin, 2 Ck5, 3 Ck17, 4 Ck20, 5 UPK3A and 6 Dapi.
Figures 7 to 10 show immunofluorescence performed on BLHOU-40 tissues, 1) healthy tissue, 2) cancer tissue.

Methods for culturing epithelial stem cells from a variety of tissues have previously been described in WO2010/090513, WO2012/014076 and WO2012/168930. However, in the case of bladder, it is very difficult to obtain organoids from muscle invasive bladder cancer as well as reproducible organoids.

The bladder is an organ whose role is to store and evacuate urine excreted by the kidneys. It is subdivided into three main layers: the mucosa, the submucosa, and the muscle. The mucosa membrane is a layer of cells that lines the inside of the bladder. It is also called urothelium or transitional epithelium. The submucosa is a layer of connective tissue that separates the mucosa and the muscularis. It is also called Lamina Propria, sub-urothelial tissue or stroma. The muscularis consists of two layers of muscle tissue, extending to the outside of the submucosa. This third layer is composed by smooth muscle cells divided into muscular longitudinal and muscular external.

The urothelium is an epithelium composed of 3 cellular subtypes: superficial cells, intermediate cells and basal cells. These cell subtypes express different levels of cytokeratin (Ck 5, 7, 8, 10 and 20). The basal cells express a high level of cytokeratin 5 (Ck5), and Ck17 while the cells in umbrellas (superficial cells) express high levels of Ck7, Ck20 and Uroplakin 3. Ck 5, 17 and p63 are markers of basal and intermediate cells whereas Ck 7, 8, 18 and 20 as well as uroplakins are markers of cells in umbrella.

The urothelium in homeostatic conditions regenerates in 3 to 6 months but is able in case of aggression to regenerate quickly in a few days. The stem cells are located at the level of the basal cell layer with an estimated proportion of 9%.

The present inventors have surprisingly found that adding a supernatant of fibroblasts to the culture medium allows human epithelial stem cells to be cultured without any defect.

This enables a large number of cells and organoids to be available for various applications, for example, drug screening, in which a large amount of material is required to test various different drugs. The ability to generate the cells from a single starting source is advantageous for such applications where it is necessary to compare results between experiments. Similarly, it means that many cells would be available for use in transplants and that multiple patients may be transplanted with cells obtained from a useful donor. Culturing the cells in an expansion medium allows the cells to multiply whilst retaining their stem or progenitor cell phenotype. Organoids are formed comprising these stem or progenitor cells.

Accordingly, there is provided a method for culturing epithelial stem cells, wherein said method comprises culturing one or more epithelial stem cells in contact with an extracellular matrix in the presence of an expansion medium, the expansion medium comprising a basal medium for animal or human cells to which is added a bovine pituitary extract (BPE), a tyrosine kinase ligand, a supernatant of fibroblasts and optionally, a ROCK inhibitor.

As explained above, long-term culture of human cells, in particular bladder cells, was very difficult prior to the present invention. Now, using the present invention, it is possible to culture bladder cells to obtain reproducible organoids.

The bladder epithelial stem cells to be cultured in the expansion method may be obtained by any suitable method. Preferably they are obtained from a human bladder and so are human bladder epithelial stem cells. In another embodiment, they can also be obtained from patient -derived - xenografts (PDX) from bladder cancer.

The bladder cells for use in the methods of invention are isolated from a bladder biopsy. In some embodiments, bladder cells are isolated by collagenase digestion, for example, as described in Southgate et al., (Culture of Epithelial Cells, 2002, Second Edition, Wiley-Liss, Inc.).

More preferably, the bladder fibroblasts come from biopsy.

The epithelial stem cells of the invention are epithelial cells from epithelial tissue of bladder or urothelial tissues.

In the expansion medium of the invention, any suitable bovine pituitary extract (BPE) can be used. Preferably, the BPE is used at a concentration comprised between 20 and 100 ng/mL of medium. More preferably, the concentration of BPE in the medium is 50 ng/mL of medium.

Concerning the Receptor Tyrosine Kinase Ligands, either Epidermal growth factor (EGF), fibroblast growth factor (FGF) or hepatocyte growth factor (HGF) are possible in the expansion medium. Many receptor tyrosine kinase ligands are mitogenic growth factors. In some embodiments, the receptor tyrosine kinase ligand in the expansion medium is selected from the group consisting of: FGF, HGF and EGF. Preferably, the receptor tyrosine kinase ligand used is EGF. Any suitable EGF may be used, for example, EGF obtained from Peprotech. Alternatively, EGF is substituted with an alternative compound that activates the EGF receptor. For example, it is envisaged that IGF (Insulin-like growth factor) may be substituted for EGF.

During culturing of stem cells, said receptor tyrosine kinase ligand is preferably added to the culture medium when required, for example, daily or every other day.

The basal medium may be supplemented with 4 ng/ml to 10 ng/ml EGF. More preferably the basal medium may be supplemented with 5ng/ml of EGF.

ROCK inhibitor is selected from the group consisting of Fasudil, Ripasudil, Netarsudil, RKI-1447, Y-27632, GSK429286A, C21H16F4N4O2 or Y-30141. Preferably, the ROCK inhibitor is Y-27632. More preferably, the medium may be supplemented with 10 µM of a ROCK inhibitor.

The supernatant of fibroblast added is preferably a supernatant of bladder primary fibroblasts obtained after 3 days to 3 weeks of culture of the cells (ref PCS-420-013) in a fibroblast basal medium (ref: PCS-201-030) completed by a fibroblast growth kit-low serum (ref: PCS-201-041).

Usually, from 30 to 60% of supernatant of fibroblasts is added to the complete KSFM medium. Preferentially, 50% of supernatant of fibroblasts is added to 50% of KSFM medium.

Some embodiments of the invention comprise the use of expansion medium for around 8-50, for example, 10-50, 15-50, 20-50, 20-40 passages of the cells. For example, in some embodiments the cells may be split at a 1:1 to 1:3 dilution every 7-10 days for 2, 3, 4, 5 or 6 or more months.

Such dilutions are needed to favor asymmetrical divisions of the stem cells and differentiated epithelial cells. Preferably, during asymmetrical divisions, the medium contains a ROCK inhibitor.

Preferably the cells are human cells. More preferably, the epithelial stem cells are human epithelial stem cells. However, culturing non-human mammalian epithelial stem cells is also envisaged.

In some embodiments, the stem cells are selected from bladder, pancreas, intestine (e.g. small intestine or colon), stomach, prostate, kidney, lung, breast, ovarian, salivary gland, hair follicle, skin, oesophagus and thyroid epithelial stem cells. In a preferred embodiment, the stem cells come from bladder cell.

As described above, the method for culturing epithelial stem cells comprises culturing one or more epithelial stem cells embedded with an extracellular matrix. Any suitable extracellular matrix may be used. Isolated epithelial stem cells are preferably cultured in a microenvironment that mimics at least in part a cellular niche in which said stem cells naturally reside. This cellular niche may be mimicked by culturing said stem cells in the presence of biomaterials, such as an extracellular matrix that provides key regulatory signals controlling stem cell fate.

A cellular niche is in part determined by the stem cells and surrounding cells, and the extracellular matrix (ECM) that is produced by the cells in said niche. In a preferred method of the invention, epithelial stem cells are cultured in contact with an ECM. "In contact" means a physical or mechanical or chemical contact, which means that for separating said resulting organoid or population of epithelial stem cells from said extracellular matrix a force needs to be used. Preferably, the epithelial stem cells are embedded in the ECM.

A culture medium of the invention may be diffused into an extracellular matrix (ECM). In a preferred method of the invention, isolated tissue fragments or isolated epithelial stem cells are attached to an ECM. ECM is composed of a variety of polysaccharides, water, elastin, and glycoproteins, wherein the glycoproteins comprise collagen, entactin (nidogen), fibronectin, and laminin. ECM is secreted by connective tissue cells. Different types of ECM are known, comprising different compositions including different types of glycoproteins and/or different combination of glycoproteins. Said ECM can be provided by culturing ECM-producing cells, such as for example fibroblast cells, in a receptacle, prior to the removal of these cells and the addition of isolated tissue fragments or isolated epithelial stem cells. Examples of commercially available extracellular matrices are extracellular matrix proteins (Invitrogen) and basement membrane preparations from Engelbreth-Holm-Swarm (EHS) mouse sarcoma cells (e.g. Matrigel™ (BD Biosciences) which comprises laminin, entactin, and collagen IV.).

The use of an ECM for culturing stem cells enhanced long-term survival of the stem cells and the continued presence of undifferentiated stem cells. In addition, the presence of an ECM allowed culturing of three-dimensional tissue organoids, which could not be cultured in the absence of an ECM.

In some embodiments, the single stem cell, population of cells, or tissue fragment is embedded in matrigel, which is optionally growth factor reduced and/or phenol red-free. In some embodiments, the expansion culture medium is placed on top of the ECM. The expansion culture medium can then be removed and replenished as and when required. In some embodiments, the culture medium is replenished every 1, 2 or 3 days. If components are "added" or "removed" from the media, then this can in some embodiments mean that the media itself is removed from the ECM and then a new media containing the "added" component or with the "removed" component excluded is placed on the ECM.

In some embodiments, the method comprises culturing the organoid or a population of epithelial stem cells for at least 2, 4, 6, 8, 10, 12, 14, 16, 18, 20 or 25 weeks.

The expansion culture medium preferably induces the survival of stem cells and their asymmetrical division. During this phase, the expansion medium contains at least the complete medium and Y27632.

The expansion medium preferably induces or promotes the survival and/or proliferation of cells during at least 1 to 2 weeks of culture. Proliferation can be assessed using techniques known in the art such as BrdU staining, Edu staining, Ki67 staining and the use of growth curves assay can be done as well as differentiation labelling assays. Morphological characterization of organoid cultures can also be assessed microscopically and with cell differentiation labelling assays.

Media used according to the invention are capable of expanding a population of stem cells to form organoids for at least 2, at least 4, at least 6, at least 8, or at least 10 passages under appropriate conditions. Preferably, at this stage, the expansion culture medium does not contain a ROCK inhibitor.

In a preferred embodiment, the epithelial stem cells to be cultured in the expansion method and/or from which the organoids are derived are obtained from adult tissue, i.e. the epithelial stem cells are adult epithelial stem cells. In this context "adult" means mature tissue, i.e. includes newly-born baby or child but excludes embryonic or foetal.

Cells taken directly from live tissue, i.e. freshly isolated cells, are also referred to as primary cells. In some embodiments the epithelial stem cells are primary epithelial stem cells. Primary cells represent the best experimental models for *in vivo* situations. In a preferred embodiment of the invention, the epithelial stem cells are (or are derived from) primary epithelial stem cells. Primary cell cultures can be passaged to form secondary cell cultures.

The epithelial stem cells may be obtained by any suitable method. In some embodiments, cells are isolated by collagenase digestion. In some embodiments, collagenase digestion is performed on a tissue biopsy.

In some embodiments, the method comprises culturing a fragment of tissue which comprises epithelium. In some embodiments, the epithelial stem cells are isolated from a tissue fragment. An organoid is preferably obtained using a cell from an adult tissue, preferably an epithelial stem cell from an adult tissue. In some embodiments, the epithelial stem cells are normal cells. In alternative embodiments, the epithelial stem cells are cancer stem cells. Accordingly, the cells may be obtained from a tumour, if required.

In a further aspect, there is provided a method for obtaining an organoid comprising culturing epithelial stem cells in an expansion medium using a method as described herein.

In some embodiments, the method comprises culturing the epithelial stem cells or obtaining the organoid/population of adult epithelial stem cells from a single cell. Advantageously, this allows a homogenous population of cells to form.

Any one of a number of physical methods of separation known in the art may be used to select the cells of the invention and distinguish these from other cell types. Such physical methods may involve FACS and various immuno-affinity methods based upon makers specifically expressed by the cells of the invention.

In a preferred embodiment, a cancer organoid obtained from cancer stem cells is grown in a culture medium that is suitable for growth of the corresponding normal tissue organoid obtained from normal stem cells, optionally with certain factors excluded from the medium. The normal tissue medium should allow cancers with all genetic backgrounds to grow, without excluding any particular cancer mutations.

The invention provides an organoid or a population of epithelial stem cells obtainable or obtained by a method of the invention. Thus, in some embodiments, the method further comprises obtaining and/or isolating an organoid.

An organoid is therefore obtainable or obtained by the method of the invention. Preferably, this organoid is derived from the bladder. More preferably, this organoid originates from non-papillary tumour.

Alternatively, an organoid is obtainable or obtained by the method of the invention is derived from a human's bladder tumour grafted into a mouse, preferably humanized, previously engrafted with bladder cancer cells from a human and containing a tumor formed from the bladder cancer cells.

It is to be understood that in a preferred expansion organoid, the majority of cells are expanding cells (i.e. dividing cells) that retain an undifferentiated phenotype. Image analysis may be used to assess characteristics of cells in culture such as cell morphology; cell structures and organoid composition and structure. Many types of imaging analysis are well known in the art, such as electron microscopy, confocal microscopy, stereomicroscopy, fluorescence microscopy.

An expansion organoid of the invention preferably has a three-dimensional structure, i.e. the organoid is preferably a three-dimensional organoid. Morphologically, the cells appear like their corresponding *in vivo* tissue counterpart. The organoid or population of epithelial stem cells may be from any mammalian tissue. In some embodiments, it is from a mouse, rabbit, rat, guinea pig or other non-human mammal. Preferably, the organoid appears like a bladder tissue, more preferably, the cells are human cells.

Within the context of the invention, a tissue fragment is a part of an adult tissue, preferably a human adult tissue, more preferably, a bladder tissue. Preferably an organoid as identified herein is therefore not a tissue fragment.

Illustrative examples of organoids generated using the differentiation medium and methods of the invention are given in the accompanying figures. The method of the invention may comprise changing the expansion medium for fresh medium during the course of the culturing because the components of the medium are used up during culturing. It will be clear to the skilled person how often the medium needs to be changed for fresh medium. In some embodiments, the medium is changed every other day, but it is also envisaged that it may be changed every day or every two days or as required.

The methods for culturing epithelial stem cells and/or obtaining an organoid in expansion or differentiation medium are carried out *in vitro.*

The invention provides an expansion medium, comprising a basal medium for animal or human cells to which is added a bovine pituitary extract (BPE), a receptor tyrosine kinase ligand, a supernatant of primary fibroblasts and optionally a ROCK inhibitor

Preferably, in the expansion medium of the present invention, the ROCK inhibitor is selected from the group consisting of Fasudil, Ripasudil, Netarsudil, RKI-1447, Y-27632, GSK429286A, C21H16F4N4O2 or Y-30141. More preferably, the ROCK inhibitor is Y-27632.

Preferably, in the expansion medium, the receptor tyrosine kinase ligand is selected from the group consisting of: FGF, HGF and EGF and more preferably is EGF.

The expansion medium of the invention contains the supernatant of primary fibroblasts. Preferably, the supernatant is a supernatant of bladder fibroblasts.

The expansion medium used in the method of the invention comprises any suitable basal medium. A basal medium for use in the invention will generally comprises a nutrient solution comprising standard cell culture ingredients, such as amino acids, vitamins, lipid supplements, inorganic salts, a carbon energy source, and a buffer, as described in more detail in the literature and below. In some embodiments, the culture medium is further supplemented with one or more standard cell culture ingredient, for example selected from amino acids, vitamins, lipid supplements, inorganic salts, a carbon energy source, and a buffer. The skilled person will understand from common general knowledge the types of culture media that might be used as the basal medium in the cell culture mediums of the invention. Potentially suitable cell culture media are available commercially, and include, but are not limited to, Dulbecco's Modified Eagle Media (DMEM), Minimal Essential Medium (MEM), Knockout-DMEM (KO-DMEM), Glasgow Minimal Essential Medium (G-MEM), Basal Medium Eagle (BME), DMEM/Ham's F12, Advanced DMEM/Ham's F12, Iscove's Modified Dulbecco's Media and Minimal Essential Media (MEM), Ham's F-10, Ham's F-12, Medium 199, RPMI 1640 Media and KSFM Media.

The invention provides a composition comprising an expansion medium of the invention, an extracellular matrix and epithelial stem cells of the invention. The invention also provides a composition comprising an expansion medium of the invention, an extracellular matrix and one or more organoids of the invention.

The organoids of the invention are used in drug discovery development; drug screen; target screen; biomarker screen; toxicity assay; gene expression studies including recombinant gene expression; research of mechanisms involved in tissue injury and repair; research of inflammatory and infectious diseases; research on the microbiota and associated dysbiosis and pathologies; studies of pathogenic mechanisms; or studies of mechanisms of cell transformation and aetiology of cancer; precision medicine and/or as *ex vivo* cell/organ models, such as disease models.

The organoids of the invention or a cell derived from said organoids can be used to treat a disorder, condition or disease. In particular, these organoids or a cell derived from these organoids are useful to treat bladder cancer, bladder inflammation, cystitis, overactive bladder, interstitial cystitis, chemical cystitis, neurogenic bladder, bladder outlet obstruction, underactive bladder or any bladder pathologies.

The organoids of the invention or a cell derived from said organoids are useful for its use in transplantation of the organoid or cell into a patient.

The organoids of the invention can also be grafted in animal models.

Cells and organoids cultured according to the media and methods of the invention are thought to faithfully represent the *in vivo* situation. This is true both for expanded populations of cells and organoids grown from normal tissue and for expanded populations of cells and organoids grown from diseased tissue. Therefore, as well as providing normal *ex vivo* cell/organ models, the organoids of the invention can be used as *ex vivo* disease models.

Organoids of the invention are also used for culturing of a pathogen and thus can be used as *ex vivo* infection models. Examples of pathogens that may be cultured using an organoid of the invention include viruses, bacteria, prions or fungi that cause disease in its animal host. Thus, an organoid of the invention can be used as a disease model that represents an infected state. In some embodiments of the invention, the organoids can be used in vaccine development and/or production. Diseases that can be studied by the organoids of the invention thus include genetic diseases, metabolic diseases, pathogenic diseases, inflammatory diseases and functional diseases.

The ability to obtain a useful organoid of the invention in short time periods shows that the organoids is highly useful for testing individual patient responses to specific drugs and tailoring treatment according to the responsiveness. Wherein the organoid is obtained from a biopsy from a patient, the organoid is cultured for less than 21 days.

The added advantage of using the organoids for identifying drugs in this way is that it is also possible to screen normal organoids (organoids derived from healthy tissue) to check which drugs and compounds have minimal effect on healthy tissue. This allows screening for drugs with minimal off-target activity or unwanted side-effects.

Therefore, the present invention encompasses a method of diagnosing a disease in a patient, comprising obtaining epithelial cells from the patient and culturing said epithelial cells *in vitro* according to the present invention and testing said cultured cells or organoid for the disease.

Moreover, there is provided a method of use of an organoid of the invention for the selection of therapeutic compounds (specific drugs) for a patient. This method comprises the steps of obtaining a biopsy of bladder cells of a patient, culturing said cells *in vitro* according to the present invention and testing potential therapeutic molecules for this patient.

This method of selection of therapeutic compounds allows the inclusion of patients in clinical trials, based on the tested therapeutic molecules.

The methods of the present invention allow a great number of organoids and epithelial stem cells to be generated in a short period of time, thereby ensuring that sufficient cells are available for use in the application of interest.

The following examples are offered for illustrative purposes only, and are not intended to limit the scope of the present invention in any way.

### Example 1: Studied Population

Organoid cultures of human bladder were performed from bladder specimens collected intraoperatively during cystectomy for urothelial bladder cancer of human patients from 50 to 70 years old.

After extraction of the surgical specimen, a vertical incision was made on the anterior surface of the bladder and two samples were taken: One in a macroscopically "healthy" zone (control group - culture A) and one in a macroscopically "tumoral" zone (tumor organoids - culture B). Two tumors were classified as muscle invasive (at least pT2 or more) and poorly differentiated (high grade) and two were non-invasive (pTl) after cystectomy and anatomo-pathological analysis of the tumour (according to the 1973 and 2004 WHO grading classification).

| Sample | Number of TR | BCG | Chemotherapy | Type of chemotherapy | Histology of TR | Histology after cystectomy |
|---|---|---|---|---|---|---|
| BLHOU-33 | 1 | 0 | 1 | MVAC | >pT2 + CIS | pT3a |
| BLHOU-34 | 1 | 0 | 1 | MVAC | pT2 G3 | pT3b |
| BLHOU-36 | 3 | 1 | 0 | 0 | pT2 | pT1 |
| BLHOU-40 | 1 | 0 | 1 | MVAC | pT4 | pT1 |

| | | | | | | |
|---|---|---|---|---|---|---|
| MVAC = Methotrexate, Vinblastin, Adriamicin, Cisplatin BCG = Bacille of Calmette and Guerin (0 = no instillation / 1 = a series of 6 instillations in the bladder) CIS = cisplatine TR = Transuretral Resection of the bladder | | | | | | |

### Example 2 Obtention of bladder cells

The bladder samples were dissected to eliminate adipose and connective tissue and to retain the urothelium and the submucosa. A "stripping" solution (Southgate et al., 2002, Culture of Epithelial Cells, Second Edition) is injected into the submucosa. The urothelium is incubated in the "stripping solution" at 4 °C overnight, with gentle shaking to break the intercellular junctions. The next day, the urothelium is removed from the muscularis / stroma using dissecting forceps and placed in collagenase IV solution (100U / ml) for 20 minutes at 37 °C. Then, to stop the dissociation, 3ml of KSFM medium (Keratinocyte Serum-Free Medium - Thermo Fischer) supplemented with Y27632 (10 µM - Sigma Aldrich, France) is added. Following strong manual agitation, the supernatant enriched in urothelial cells is centrifuged at 250 x g for 5 minutes at 4 ° C. The supernatant is discarded and the pellet containing urothelial cells resuspended in KSFM supplemented medium containing 50 ng/mL of BPE and 5ng/mL of EGF. After centrifugation and suspension in a volume of Matrigel (Matrigel hESC-qualified Matrix, BD Biosciences) at 4 °C, a concentration of 2 x 10⁶ cells /mL of Matrigel is obtained.

### Example 3: Culture cells in slides

8-well Lab-teks (Lab-tek II Chamber Slides system, Dutscher) or 96-well µ-angiogenesis-Ibidi plates are used (60 000 cells in 25 µL of Matrigel for 8-well labteck and 25 000 cells seeded in 10 µL, of Matrigel for 96-well plate). Matrigel is polymerized for 20 minutes at 37 °C and then the complete medium is added (one volume of KSFM supplemented medium and one volume of primary fibroblast supernatant (PFS) isolated from the culture of human bladder fibroblasts). The supernatant of fibroblasts is obtained after 3 to 4 days of culture of the fibroblasts (ATCC- PCS-420-013) in a fibroblast basal medium (ATCC- PCS-201-030) completed by a fibroblast growth kit-low serum (ATCC- PCS-201-041). During the first 3 days of culture, this complete expansion medium is supplemented with Y27632 (10 µM). The medium is changed every 2-3 days with fresh expansion medium which corresponds to the complete medium without Y27632 (250 µL / well in the 48 well plates and labtek, 100 µL / well in the 96-well plates). Plates are incubated at 37 °C in normoxia condition (5% CO2).

### Example 4: Morphological studies of "healthy" organoids

### a) Comparison of culture media:

Culture wells of healthy control cells (A cultures) are made with and without PFS in the culture. Then, the cells are monitored and compared at day 14.

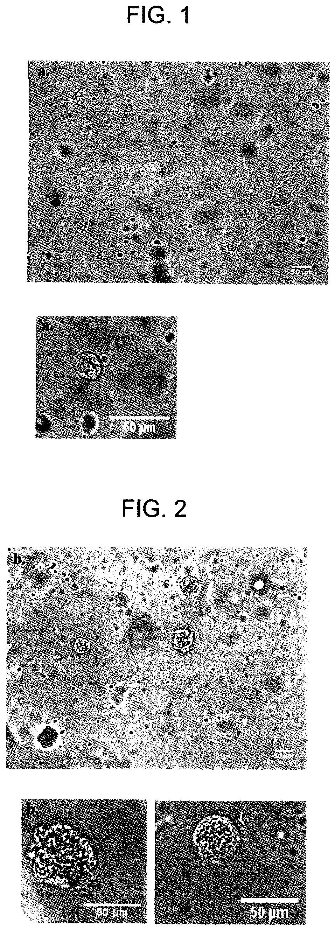

A larger number of structures as well as a substantially greater diameter for the organoids subjected to a culture medium supplemented with PFS are observed at D14 (FIG. 2) (approximately 40 organoids per well of 80 µm against a dozen structures of 20 microns in diameter with the KSFMc (Fig 1)). This was observed with all the cultures of organoids performed.

The culture medium supplemented with primary fibroblast supernatant (PFS) thus seemed to promote the proliferation and growth of organoids compared to the KSFM culture medium complemented with EGF and BPE alone.

### b) Growth monitoring of organoids:

A total of 6 cultures of healthy organoids were carried out with the culture medium supplemented by PFS. We then compared the median organoid areas (solid structures) for these cultures at 14 days. There was a significant increase in the median organoid area on Day 14 (p = 0.0013).

### Example 5: Comparative study of the morphology of organoids from healthy and tumour bladders.

The growth and morphology of organoids derived from patients with healthy control organoids (A cultures) or bladder cancer (B cultures) were monitored at day 7.

The observations were made under a wide-field microscope at 10x magnification (Apotome Zeiss, 10x objective). When acquiring images, the choice of fields was randomly made. The size of the organoids has been evaluated using the image processing software Image J.

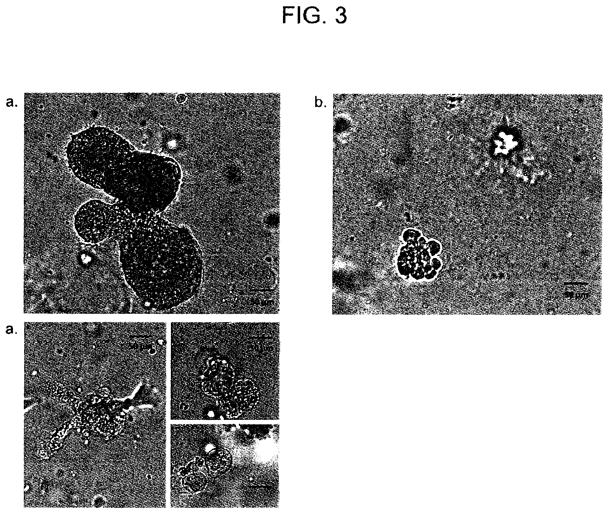

In tumor cultures, solid organoids classically observed in healthy cultures but also new morphologies were found. Indeed, the new solid structures for tumor cultures can be classified as full multilobed organoids, "bilobed" (Figure 3a) or "in clusters" (Figure 3b). These different forms have never been observed in healthy cultures.

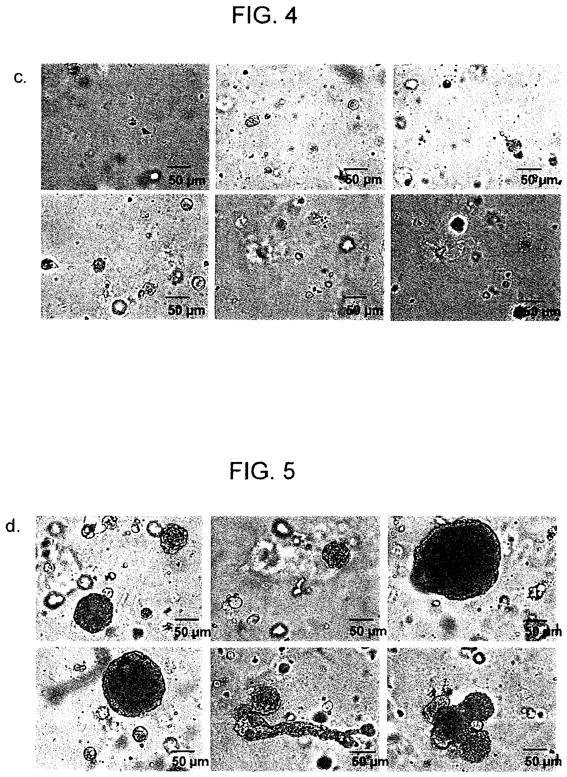

The size of the tumor organoids appeared to be higher than the healthy organoids, as shown between the healthy (A) and tumor (B) cultures (Figures 4 and 5). The comparison was made measuring 340 organoids, 170 healthy and 170 tumours.

### Example 6: Immunolabeling of organoids

### - Immunolabeling on tissue sections

Immunolabeling is performed on frozen bladder sections. Fresh samples were included in OCT (Optimal Cutting Temperature), cut longitudinally, and immediately frozen on dry ice and stored at -80 °C. They are then cut with cryostat in 5 µm sections, deposited on slides (Superfrost Plus, Thermo Scientific) and fixed with 3.7% formaldehyde for 20 minutes at room temperature or with 50% acetone / 50% methanol until evaporation, depending on the antibody used. Blocking of nonspecific antigenic sites and permeabilization were achieved by a solution containing PBS / 1% BSA / 0.5% Triton X100. Samples are incubated with diluted primary antibodies in blocking buffer overnight at 4 °C in a humid chamber. After washing with PBS without calcium / magnesium IX, they are incubated for 1 hour with the secondary antibodies in the dark, at room temperature. The different antibodies used are summarized in Table 1 below. DAPI (4',6-diamidino-2-phenylindole) nuclei labeling (diluted 1/200, incubated 20 min at room temperature) is performed and the slides are mounted with a drop of Prolong gold mounting medium and a coverslip.

**Table 1: Antibodies used for immunolabeling**

| Antibody | Species | Ref Provider | Dilution | Tissue fixation |
|---|---|---|---|---|
| Cytokeratin 5 | Rabbit | ref 905501 Biolegend | /100 | 50/50 acetone methanol |
| Cytokeratin 17 | Rabbit | Ref ab10972 5Abcam | /100 | 50/50 acetone methanol |
| Cytokeratin 20 | Mouse | Ref M7019 Dako | /50 | FA 3.7% |
| Uroplakine 3a | Rabbit | Ref abin1688372 Santa cruz | /50 | 50/50 acetone methanol |
| GATA 3 | Rabbit | Ref AAB 106625 Abcam | /800 | Formaldehyde 3.7% |
| Ki 67 | Mouse | Ref M7240 Dako | /100 | Formaldehyde 3.7% |
| Alexa A568 anti mouse | Donkey | Ref A10037 Life Technologies | /500 | |
| Alexa A555 anti rabbit | Donkey | Ref A31572 Life technologies | | |

### - Immunolabeling on organoids

Organoids are fixed at culture day 7 or 14 with 3.7% formaldehyde for 40 minutes (labteks) or 20 minutes (96-well) at room temperature. They are permeabilized with 0.5% Triton (in PBS) and the aspecific antigenic sites are blocked for 1.5 hours at 37 °C with a 3% BSA solution in PBS. Then the organoids are incubated with the primary antibodies at 4°C overnight followed by one hour at 37 °C. After washes, the secondary antibodies are incubated for one hour at 37 °C in the dark. DAPI nuclei labeling (dilution 1/200e) and actin labeling by Alexa Fluor-488 phalloidin (diluted 1: 500 in DAPI solution) is performed. After incubation for 20 minutes in the dark at room temperature, washes are performed and a drop of Vectashield mounting medium (Vector Labs) without DAPI is deposited in each well.

The organoids and slides were observed using the Zeiss LSM 710 confocal microscope with 20x and 40x objectives. For each experiment, sections or control wells are imaged either without any antibodies (control of autofluorescence) and with the secondary antibodies alone. The control images were acquired with the same microscopy parameters and processed identically using the Image J software.

The organoids were also imaged with an automated imaging device (Opera Phenix, Perkin Elmer) with 20x water objectives. The images were processed with the Harmony software that drives the device.

### Example 7: Characterization of healthy organoids by immunofluorescence (IF)

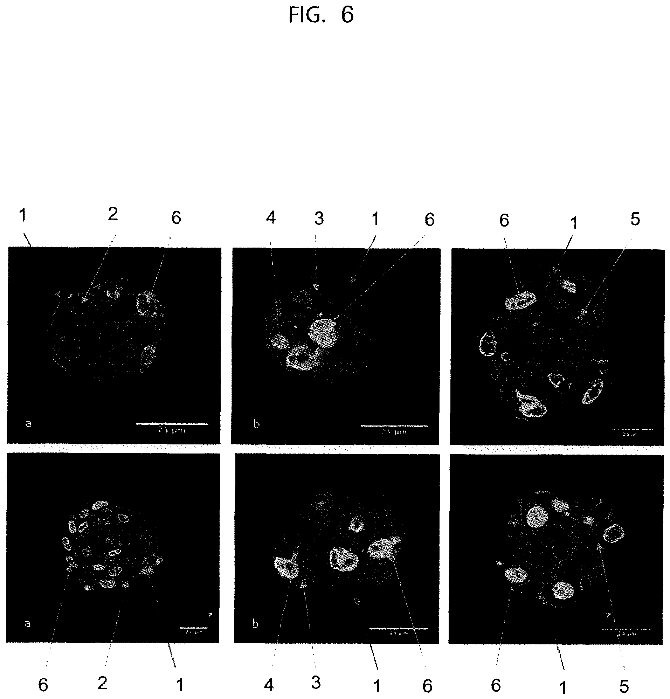

These structures were observed using a Zeiss LSM 710 confocal microscope at day 14. The epithelial markers Ck5 and Ck17 are physiologically expressed in undifferentiated basal cells. Ck20 is a predominant epithelial marker on superficial, differentiated cells. There was then a peripheral disposition of undifferentiated cell markers with a predominance of Ck20 at the center of the structures. In addition, Uroplakin-3A spots are visualized in the center of the structures. Uroplakin-3A (UPK3A) is a protein that plays a major role in urothelial barrier function and is therefore characteristic of differentiated cells. Cell differentiation therefore seems hierarchical, occurring from the outside to the inside of these organoids (see Figure 6).

### Example 8: Characterization of tumor tissues and organoids by immunofluorescence

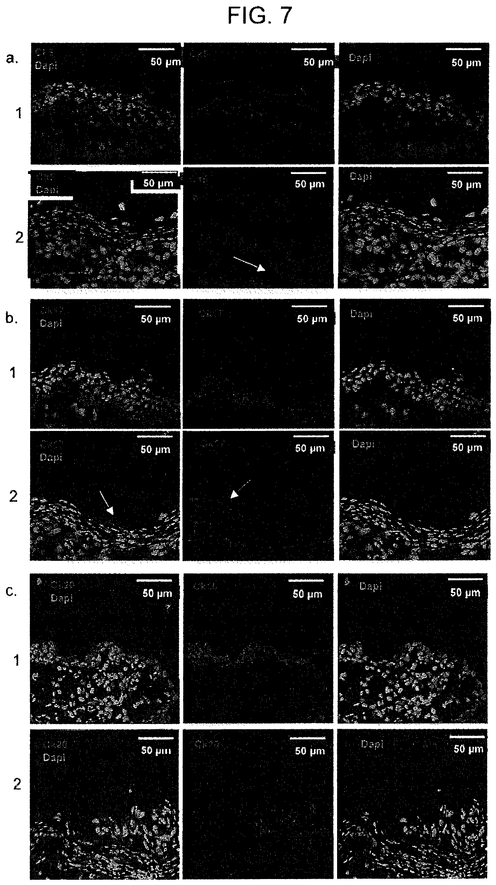

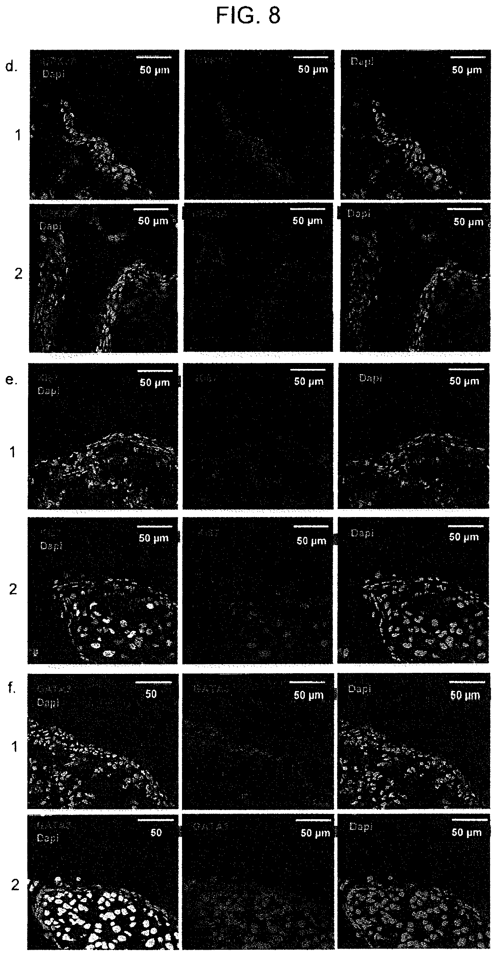

We performed labeling on sections of healthy bladders and tumours with different urothelial markers (Figures 7 and 8). We found a similar distribution of these markers on samples A and B. Indeed, Ck17 was found preferentially at the level of the basement membrane and Ck20 in contact with the light (here facing upwards images). There was, however, unusual uptake of Ck17 in the superficial cells for tumor cutting (indicated by a white arrow). In the same way, there was an unusual fixation of Ck5 at the submucosal level on the tumor section (indicated by a white arrow). Uroplakin-3A was also observed in superficial cells in contact with bladder light. The tumor tissue sections used for labeling Ki67 (cell proliferation marker) and GATA3 (nuclear transcription factor) were probably located at the level of a papillary fringe, as evidenced by the histological aspect and the thickness of the urothelium. Overexpression of Ki67 and GATA3 was observed on these tumor sections, evoking an increase in cell proliferation.

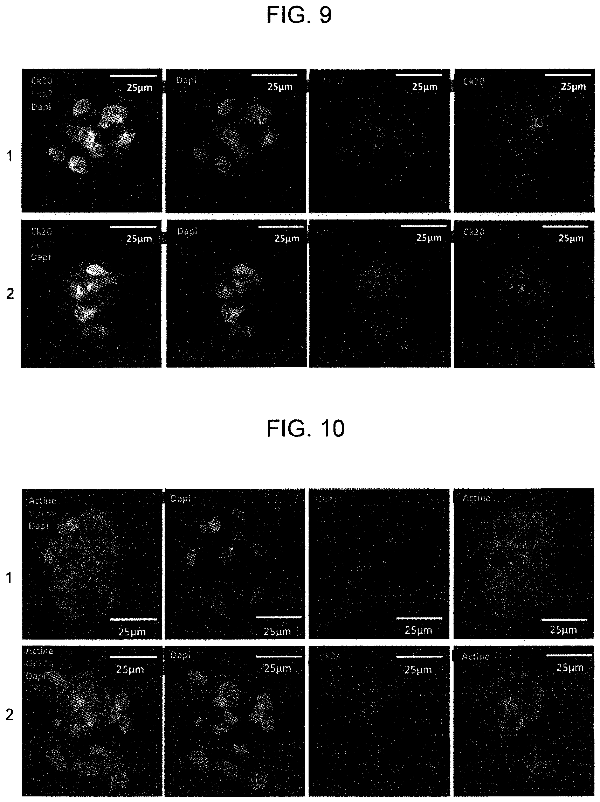

The organoids were then observed on day 14 under a confocal microscope, where IF labeling was performed with the same antibodies as those used on tissue sections (FIG. 9 and 10).

We then observed an expression of the different urothelial markers, including Upk3A, showing differentiated cell expression and confirming the urothelial character of these structures. It seems that cell differentiation is from the outside to the inside of the structure. Indeed, the Ck17 (basal marker) was observed mainly at the periphery of the structure while Ck20 or UPK3A (luminal markers) were expressed towards the center of these organoids whether in healthy cultures or tumours.

There is no specific difference between the IF labeling of organoids according to the healthy or tumor character under a confocal microscope.

### Example 9: Pharmacological Responses of organoids following treatments

The organoids were cultured in Matrigel in 96-well black plates for 15 days. Healthy and tumorous organoid cultures were treated with cisplatin and gemcitabine from the 8th day of culture. The concentrations used are 2 µM, 20 µM and 200 µM for cisplatin and 0.05 µM, 0.5 µM and 5 µM for gemcitabine. These molecules were added to the culture medium, the latter being changed every two days until the 15th day of culture. The gemcitabine and cisplatin are chemotherapy medications used to treat bladder cancers (Rouprêt et al., Progrès en Urologie, 2018, 28:S46-S78).

The cell viability was analyzed using the CellTiter-Glo®3D kit (Promega, # 9683) at the fifteenth day of culture. Briefly, in order to dissociate the organoids, they were incubated with Tryple Express solution (Gibco) for 40 minutes at 37°C by pipetting regularly. After dissociation of the structures, the CellTiter-Glo®3D reagent was added to the culture medium of each well (volume 1:1) and incubated for 30 minutes at room temperature, in the dark, with stirring, according to the manufacturer's instructions. Luminescence was measured with a luminometer (Varioskan Flash-Thermo Scientific, Illkirch, France). Three wells per condition were measured and analyzed. Data analyzes were performed using Excel software, and JC50 values were calculated using non-linear regression. Comparisons between groups were made using the paired or unpaired Student t-test. The level of significance retained was p <0.05. Statistical analysis was performed using PRISM (Graph Pad Prism® Version 5.0, Software, Inc., California, USA).

The IC50 of cisplatin for tumor culture was 1.75µM while the IC50 for healthy culture was 4.2µM (factor 2.4). These results show a greater effectiveness of treatment on tumor cells for the same concentration. The IC50 of Gemcitabine for tumor culture was 1.2 µM while the IC50 for healthy culture is 2.8 µM, also demonstrating a higher efficiency of chemotherapy on tumor culture (factor 2.3).

### Example 10: Organoids from PDX

Organoids are developed from either fresh or frozen PDX obtained from bladder cancer. To obtain the organoids, the PDX tissue is handled as if it was the tumor tissue of a patient according to the protocol of example 2. Then, the same protocol as in examples 3, 5, 6, 8 and 9 is used to derive and obtain the organoids from the PDX.

## Claims

1. A method for differentiating epithelial stem cells, wherein said method comprises: culturing one or more epithelial stem cells in contact with an extracellular matrix in the presence of an expansion medium, the expansion medium comprising a basal medium for animal or human cells to which is added a bovine pituitary extract (BPE), a receptor tyrosine kinase ligand, a supernatant of primary fibroblasts, and optionally, a Rho kinase inhibitor (rho-associated protein kinase inhibitor or ROCK inhibitor).

2. A method according to claim 1, wherein, in the expansion medium, the receptor tyrosine kinase ligand is selected from the group consisting of FGF, HGF and EGF.

3. A method according to claim 1 or 2, wherein, in the expansion medium, the receptor tyrosine kinase ligand is EGF.

4. The method according to any of claims 1 to 3, wherein, in the expansion medium, the ROCK inhibitor is selected from the group consisting of Fasudil, Ripasudil, Netarsudil, RKI-1447, Y-27632, GSK429286A, C21H16F4N4O2 or Y-30141.

5. The method according to any of claims 1 to 4, wherein, in the expansion medium, the ROCK inhibitor is Y-27632.

6. The method according to any of claims 1 to 5, wherein the supernatant of primary fibroblasts is a supernatant of primary fibroblast originating from human bladder.

7. The method of any one of the preceding claims, wherein the epithelial stem cells are from the bladder.

8. An expansion medium, comprising a basal medium for animal or human cells to which is added a bovine pituitary extract (BPE), a receptor tyrosine kinase ligand, a supernatant of primary fibroblasts and optionally, a ROCK inhibitor.

9. The expansion medium of claim 8, wherein the ROCK inhibitor is selected from the group consisting of Fasudil, Ripasudil, Netarsudil, RKI-1447, Y-27632, GSK429286A, C21H16F4N4O2 or Y-30141.

10. The expansion medium of claim 9, wherein the ROCK inhibitor is Y-27632.

11. The expansion medium of any one of claims 8 to 10, wherein the receptor tyrosine kinase ligand is selected from the group consisting of FGF, HGF and EGF.

12. The expansion medium of any one of claims 8 to 11, wherein the supernatant of primary fibroblasts is a supernatant of bladder fibroblasts.

13. An organoid obtainable or obtained by a method of any one of claims 1 to 7, wherein the organoid is derived from the bladder

14. An organoid according to claim 13, wherein this organoid originates from non-papillary tumour.

15. An organoid obtainable or obtained by a method of any one of claims 1 to 7, wherein the organoid is derived from a human's bladder tumour grafted into the mouse.

16. Use of an organoid as defined in claim 13, or a cell derived from said organoid for drug discovery development; drug screen; target screen; biomarker screen; toxicity assay; gene expression studies including recombinant gene expression; research of mechanisms involved in tissue injury and repair; research of inflammatory and infectious diseases; research on the microbiota and associated dysbiosis and pathologies; studies of pathogenic mechanisms; or studies of mechanisms of cell transformation and aetiology of cancer; precision medicine.

17. An organoid according to claim 13 or 14, or a cell derived from said organoid, for use in treating a disorder, condition or disease.

18. An organoid according to claim 13, or a cell derived from said organoid, for use in treating bladder cancer, bladder inflammation, cystitis, overactive bladder, interstitial cystitis, chemical cystitis, neurogenic bladder, bladder outlet obstruction, underactive bladder or any bladder pathologies.

19. An organoid according to claim 13 or 14, or a cell derived from said organoid, for use in transplantation of the organoid or cell into a patient.

20. A method of diagnosing a disease in a patient, comprising obtaining epithelial cells from the patient and culturing said epithelial cells *in vitro* according to the method of claim 1 and testing said cultured cells or organoid for the disease.

21. A method of use of an organoid according to any of claim 1 to 7 for the selection of therapeutic compounds, consisting in obtaining a biopsy of bladder cells, culturing said cells *in vitro* according to the method of claim 1 and testing potential therapeutic molecules.

22. A method of use according to claim 21, for the inclusion of patients in clinical trials, based on the tested therapeutic molecules.
